# EUROPÄISCHE PATENTSCHRIFT

(11) **EP 1 226 147 B1**
(45) Veröffentlichungstag und Bekanntmachung des Hinweises auf die Patenterteilung: **14.06.2006**
(21) Anmeldenummer: 00979510.5
(22) Anmeldetag: 25.10.2000
(51) Int. Cl.: C07F 9/145, C07F 15/04, C07C 253/10, C07C 253/30, B01J 31/18

(54) **PHOSPHITE**
PHOSPHITE
PHOSPHITE

(30) Priorität: 03.11.1999 DE 19953058
(43) Veröffentlichungstag der Anmeldung: 31.07.2002
(60) Teilanmeldung: 05024540.6
(73) Patentinhaber: BASF Aktiengesellschaft, 67056 Ludwigshafen (DE)
(72) Erfinder: BAUMANN, Robert, 68161 Mannheim (DE); FISCHER, Jakob, 85356 Freising (DE); JUNGKAMP, Tim, 69221 Dossenheim (DE); KUNSMANN-KEITEL, Dagmar, Pascale, 67117 Limburgerhof (DE); SIEGEL, Wolfgang, 67117 Limburgerhof (DE)
(86) Internationale Anmeldenummer: PCT/EP2000/010523
(87) Internationale Veröffentlichungsnummer: WO 2001/036429

(56) Entgegenhaltungen:
- WO-A-97/36856
- DE-A- 2 237 703
- US-A- 3 766 237
- CHEMICAL ABSTRACTS, vol. 61, no. 1, 1964 Columbus, Ohio, US; abstract no. 621f, ANGERT L.G.: "Synthesis of mixed esters of alpha-naphtylphosphorous acid and their inhibiting action in oxidizing rubbers" XP002161284 & ZH. PRIKL. KHIM., Bd. 36, Nr. 10, 1963, Seiten 2270-2276,

## Beschreibung

Die vorliegende Erfindung betrifft neue Phosphite, Verfahren zu ihrer Herstellung, ihre Verwendung als Ligand in Übergangsmetallkomplexen, neue Übergangsmetallkomplexe, Verfahren zu ihrer Herstellung, ihre Verwendung als Katalysator und Verfahren in Gegenwart solcher Übergangsmetallkomplexe als Katalysator.

Triarylphosphite, Nickel-Komplexe mit solchen Phosphiten als Liganden und die Verwendung solcher Komplexe als Katalysator sind bekannt.

DE-OS 2 237 703, US-A-3,850,973 und US-A-3,903,120 beschreiben ein Verfahren zur Hydrocyanierung von ungesättigten organischen Verbindungen und die Isomerisierung von Nitrilen in Anwesenheit von Nickel(0)-Komplexen mit Tri-o-tolyl-phosphit als Ligand. Nachteilig bei diesem Verfahren ist, dass die Stabilität solcher Nickel-Komplexe unbefriedigend ist. Diese geringe Stabilität zeigt sich in einem sehr geringen Gehalt an Ni(0), welches für die Hydrocyanierung die aktive Spezies ist, in der Reaktionslösung.

US-A-3,766,237 und US-A-3,903,120 beschreiben ein Verfahren zur Hydrocyanierung von ungesättigten organischen Verbindungen und die Isomerisierung von Nitrilen in Anwesenheit von Nickel(0)-Komplexen mit Tri-mfp-tolyl-phosphit als Ligand. Nachteilig bei diesem Verfahren ist, dass die Reaktivität solcher Nickel-Komplexe unbefriedigend ist.

Es war daher die technische Aufgabe gestellt, ein Verfahren zur Verfügung zu stellen, das die Hydrocyanierung von ungesättigten organischen Verbindungen bei hoher Stabilität und hoher Reaktivität des Katalysators auf technisch einfache und wirtschaftliche Weise ermöglicht.

Demgemäß wurden Phosphite der Formel I

P (O-R¹)ₓ (O-R²)_{y} (O-R³)_{z} (O-R⁴)ₚ I

Mit
- R¹:: aromatischer Rest mit einem Alkylsubstituenten aus der Gruppe n-Propyi-, i-Propyl-, n-Butyl-, i-Butyl-, s-Butyl-, n-Pentylreste sowie deren Isomeren, n-Hexylreste sowie deren Isameren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können, in o-Stellung zu dem Sauerstoffiatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
x : 1 oder 2,
y, z, p: unabhängig voneinander 0, 1 oder 2 mit der Maßgabe, daß x+y+z+p = 3,
sowie Verfahren zu ihrer Herstellung, ihre Verwendung als Ligand in Übergangsmetallkomplexen, neue Übergangsmetallkomplexe, Verfahren zu ihrer Herstellung, ihre Verwendung als Katalysator und Verfahren in Gegenwart solcher Übergangsmetallkomplexe als Katalysator gefunden.

Erfindungsgemäß ist der Rest R¹ ein aromatischer Rest mit einem Alkylsubstituenten aus der Gruppe n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können, in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System.

Als aromatischer Rest kommen Heterocyclen, vorzugsweise Homocyden, wie der Phenylrest in Betracht.

Der aromatische Rest kann weitere funktionelle Gruppen tragen, wie Alkoxy-Gruppen oder Halogene, beispielsweise Chlor oder Brom, tragen, vorzugsweise trägt der aromatische Rest keine funktionellen Gruppen.

Der aromatische Rest trägt erfindungsgemäß einen Alkylsubstituenten aus der Gruppe n-Propyl-, i-Propyl-, n-Butyl-, i-Butyl-, s-Butyl-, n-Pentylreste sowie deren isomeren, n-Hexylreste sowie deren isomeren. Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können, in o-Stellung zu dem Sauerstoffatom, das das Phosphoratom mit dem aromatischen System verbindet oder ein in o-Stellung zu dem Sauerstoffatom, das das Phosphoratom mit dem aromatischen System verbindet, anelliertes aromatisches System. Als Alkylrest können n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohoxylreste, wobei die cydischen Alkylreste lineare oder weitere cydische Alkylreste oder aromatische Reste und die Alkylreste cyclische Alkylreste oder aromatische Reste als Substituenten tragen können, eingesetzt werden. Bevorzugte Alkylreste sind n-Propyl-, i-Propyl-, n-Butyl-, i-Butyl-, s-Butyl-Gruppen.

Als aromatischer Substituent kommen Heterocyclen, vorzugsweise Homocyclen, wie der Phenylrest in Betracht.

Der aromatische Substituent kann einen oder mehrere Alkylsubstituenten aus der Gruppe n-Propyl-, i-Propyl-, n-Butyl-, i-Butyt-, s-Butyl-, n-Pentylreste sowie deren Isomeren, n-Hexylreste sowie deren lsomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können, oder frei von weiteren Substituenten sein.

Als Rest R¹ kommen vorteilhaft o-n-Propyl-phenyl-, o-Isopropyl-phenyl-, o-n-Butyl-phenyl-, o-sek-Butyl-phenyl-, o-tert-Butyl-phenyl- oder (o-Phenyl)-Phenyl-Gruppen in Betracht.

Erfindungsgemäß ist der Rest R² ein aromatischer Rest mit einem C₁-C₁₈₋Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt.

Als aromatischer Rest kommen Heterocyclen, vorzugsweise Homocyclen, wie der Phenylrest in Betracht

Der aromatische Rest kann weitere funktionelle Gruppen tragen, wie Alkvxy-Gruppen oder Halogene, beispielsweise Chlor oder Brom, tragen, vorzugsweise trägt der aromatische Rest keine funktionellen Gruppen.

Der aromatische Rest trägt erfindungsgemäß einen C₁-C₁₈-Alkylsubstituenten in m-Stellung zu dem Sauerstoffatom, das das Phosphoratom mit dem aromatischen System verbindet oder ein in m-Stellung zu dem Sauerstoffatom, das das Phosphoratom mit dem aromatischen System verbindet, anelliertes aromatisches System. Als Alkylrest können lineare oder cyclische C₁- bis C₁₈-Reste, vorzugsweise C₁-C₉-Resie eingesetzt werden, wie Methyl-, Ethyl-, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, 1-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substitierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als aromatischer Substituent kommen Heterocyclen, vorzugsweise Homocyclen, wie der Phenylrest in Betracht.

Der aromatische Substituent kann weitere funktionelle Gruppen tragen, wie Alkoxy-Gruppen oder Halogene, beispielsweise Chlor oder Brom, tragen, vorzugsweise trägt der aromatische Substituent keine funktionellen Gruppen.

Der aromatische Substituent kann einen oder mehrere C₁-C₁₈-Alkylsubstituenten oder eines oder mehrere anellierte aromatische Systeme tragen oder frei von weiteren Substituenten sein.

Als Alkylrest können lineare oder cydische C₁- bis C₁₈-Reste, vorzugsweise C₁-C₉₋Reste eingesetzt werden, wie Methyl-, Ethyl, n-Propyl-, i-Propyl-, n-Butyl-, i-Butyl-, s-Butyl-, n-Pentylreste sowie deren Isomeren, n-Hexylreste sowie deren isomeren. Cyclapentyl- oder Cyclohexylreste, wobei die cyclischen Alkylreste lineare oder weitere cyclische Alkylreste oder aromatische Reste und die Alkylreste cydische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substitierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als Rest R² kommen vorteilhaft m-Totyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen in Betracht.

Erfindungsgemäß ist der Rest R³ ein aromatischer Rest mit einem C₁-C₁₈₋Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt.

Als aromatischer Rest kommen HeterocyGen, vorzugsweise Homocyclen, wie der Phenylrest in Betracht.

Der aromatische Rest kann weitere funktionelle Gruppen tragen, wie Alkoxy-Gruppen oder Halogene, beispielsweise Chlor oder Brom, tragen, vorzugsweise trägt der aromatische Rest keine funktionellen Gruppen.

Der aromatische Rest trägt erfindungsgemäß einen C₁-C₁₈-Alkylsubstituenten in p-Stellung zu dem Sauerstoffatom, das das Phosphoratom mit dem aromatischen System verbindet oder ein in p-Stellung zu dem Sauerstoffatom, das das Phosphoratom mit dem aromatischen System verbindet, anelliertes aromatisches System. Als Alkylrest können lineare oder cyctische C₁- bis C₁₈-Reste, vorzugsweise C₁-C₉-Reste eingesetzt werden, wie Methyl-, Ethyl-, n-Propyl-, i-Propyl-, n-Butyl-, i-Butyt-, s-Butyl-, n-Pentylreste sowie deren Isomeren, n-Hexylreste sowie deren Isomeren, Cyclopentyl- oder Cyctohexyireste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-. Ethyl-, n-Propyl-, i-Propyl-, n-Butyl-, i-Butyl-, s-Butyt-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substitierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als aromatischer Substituent kommen Heterocyclen, vorzugsweise Homocyclen, wie der Phenylrest in Betracht.

Der aromatische Substituent kann weitere funktionelle Gruppen tragen, wie Alkoxy-Gruppen oder Halogene, beispielsweise Chlor oder Brom, tragen, vorzugsweise trägt der aromatische Substituent keine funktionellen Gruppen.

Der aromatische Substituent kann einen oder mehrere C₁-C₁₈-Alkylsubstituenten oder eines oder mehrere anellierte aromatische Systeme tragen oder frei von weiteren Substituenten sein.

Als Alkylrest können lineare oder cyclische C₁- bis C₁₈-Reste, vorzugsweise C₁-C₉₋Reste eingesetzt werden, wie Methyl-, Ethyl, n-Propyl-, i-Propyl-, n-Butyh, i-Butyl-, s-Butyl-, n-Pentylreste sowie deren Isomeren, n-Hexylreste sowie deren Isomeren, Cyclopentyl- oder Cydohexytreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substitierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht

Erfindungsgemäß ist der Rest R⁴ ein aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt.

Der aromatische Rest kann funktionelle Gruppen tragen, wie Alkoxy-Gruppen oder Halogene, beispielsweise Chlor oder Brom, tragen, vorzugsweise trägt der aromatische Rest keine funktionellen Gruppen.

Als Rest R⁴ kommt vorzugsweise der Phenylrest in Betracht

Von den Resten R¹, R², R³ oder R⁴ können zwei oder drei Reste in der Formel I über C₁-C₁₈ Alkylengruppen oder direkt miteinander verknüpft sein.

Als Alkylengruppen können lineare oder cydische C₁- bis C₁₈-Reste, vorzugsweise C₁₋C₉-Reste eingesetzt werden, wie Methylen-, Ethylen-, n-Propylen-, n-Butylen-, n-Pentylenreste sowie deren Isomeren, n-Hexylreste sowie deren fsomeren, Cyclopentyl-oder Cyclohexylreste, wobei die cyclischen Alkylreste lineare oder weitere cydische Alkylreste oder aromatische Reste und die Alkylreste cyclische Alkylreste oder aromatische Reste als Substituenten tragen können, wie beispielsweise in 1-Methylethylen-, 1,1-Dimethylethylen-, 1,2-Dimethylethylen-, 1-Methyl-n-propylen-, 2-Methyl-n-propylen, 1,1-Dimethyl-n-propylen-, 1,2-Dimethyl-n-propylen-, 1,3-Dimethyl-n-propylen-, 2,2-Dimethyl-n-propylen-resten.

Die Alkylenreste können weitere funktionelle Gruppen tragen, wie Alkoxygruppen, Aminogruppen, wie unsubstituierte, mono- oder disubstituierte Aminogruppen, Mercaptogruppen, wie substitierte Mercaptogruppen, wobei die Substitution durch die bei der Definition von R¹, R² oder R³ genannten Alkylgruppen oder aromatischen Reste erfolgen kann. Vorzugsweise tragen die Alkylreste keine funktionellen Gruppen.

In den Alkylenresten können Kohlenstoffatome durch andere Atome, wie Sauerstoff, Stickstoff oder Schwefel, substituiert sein, vorzugsweise sind die Alkylenreste nicht substituiert.

Erfindungsgemäß beträgt der Index x 1 oder 2.

Erfindungsgemäß betragen die Indizes y, z und p unabhängig voneinander 0, 1 oder 2 mit der Maßgabe, dass die Summe der Indizes x, y, z und p, also x+y+z+p, 3 ist.

Vorzugsweise ist p=0.

Hieraus ergeben sich folgende efindungsgemäße Möglichkeiten für die indizes x, y, z und p:

| x | y | Z | P |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Besonders bevorzugte Phosphite sind solche, in denen R¹ der o-isopropyl-Phenyl-rest, R² der m-Tolylrest und R³ der p-Tolylrest ist mit den in der Tabelle genannten Indizes, solche denen R¹ der o-Isopropylrest, R² der Z-Naphthylrest und R³ der p-Tolylrest ist mit den in der Tabelle genannten Indizes sowie Gemische dieser Phosphite.

Phosphite der Formel I können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I.

Die Umsetzung kann in drei getrennten Schritten durchgeführt werden.

Es können zwei der drei Schritte kombiniert werden, also a mit b oder b mit c.

Es können alle der Schritte a, b und c miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid CI, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCI₃.

In den Schritten a, b und c kann man die Umsetzung vorteilhaft bei Temperaturen im Bereich von 10 bis 200 C, vorzugsweise 50 bis 150 C, insbesondere 70 bis 120 C durchführen.

In den Schritten a, b und c kommt vorzugsweise ein molares Verhältnis zwischen den in den jeweiligen Schritten eingesetzten Halogenidresten und Hydroxylgruppen der eingesetzten Alkohole im Bereich von 1 : 10 bis 10:1, vorzugsweise 1 : 3 bis 3: 1, in Betracht.

Die Umsetzung in den Schritten a, b und c kann man in Gegenwart eines anorganischen oder organischen, insbesondere flüssigen, Verdünnungsmittels durchführen, wie eines Esters, beispielsweise Essigsäureethylester, eines Ethers, beispielsweise Methyl-t-butylether, Diethylether, Dioxan. Tetrahydrofuran, einer aromatischen Verbindung, beispielsweise Toluol, oder eines halogenierten Kohlenwasserstoffs, beispielsweise halogenierten Kohlenwasserstoffs, wie Tetrachlormethan, Chloroform, Methylenchlorid, oder eines Gemisches solcher Vordünnungsmittel.

Vorzugsweise führt man die Umsetzung ohne ein solches anorganisches oder organisches Verdünnungsmittel durch.

Den bei der Umsetzung entstehenden Halogenwasserstoff, der unter den Reaktionsbedingungen üblicherweise gasförmig anfällt, kann vorteilhaft abgetrennt und an sich bekannten chemischen Verfahren zugeführt werden.

Üblicherweise erhält man In den Schritten a, b und c Mischungen, die die gewünschte Komponente enthalten.

Die Abtrennung der gewünschten Komponente kann in an sich bekannter Weise, beispielsweise durch Extraktion oder Destillation, vorzugsweise durch Destillation erfolgen.

Erfolgt die Abtrennung durch Destillation, so hat sich eine Verminderung des Drucks unter Umgebungsdruck als vorteilhaft erwiesen.

Die Destillation kann vorteilhaft in einer Kolonne, beispielsweise mit Seitenabzug, oder mehreren, wie zwei, drei, oder vier Kolonnen erfolgen.

Als Kolonnen können dabei an sich bekannte Kolonnen, wie Glackenbodenkolonnen, Siebbodenkolonnen oder Füllkörperkolonnen eingesetzt werden.

Die zur Abtrennung der entsprechenden Phosphite der Formel I optimalen Verfahrensbedingungen können dabei jeweils durch wenige einfache Vorversuche leicht ermittelt werden.

Die Phosphite der Formel 1 können als Uganden in Übergangsmetallkomplexen eingesetzt werden.

Als Übergangsmetalle kommen dabei vorteilhaft die Metalle der 1. bis 2. und 6. bis 8. Nebengruppe des Periodensystems, vorzugsweise der 8. Nebengruppe des Periodensystems, besonders bevorzugt Eisen, Kobalt und Nickel, insbesondere Nickel in Betracht.

Setzt man Nickel ein, so kann dieses in verschiedenen Wertigkeiten, wie 0, +1, +2, +3, vorliegen. Bevorzugt ist hierbei Nickel(0) und Nickel(+2), insbesondere Nickel(0).

Zur Herstellung der Übergangsmetallkomplexe kann man eine ein Übergangsmetall enthaltende chemische Verbindung oder vorzugsweise ein Übergangsmetall mit einem Phosphit der Formel I um, wobei als Phosphit der Formel l ein einzelnes Phosphit der Formel oder ein Gemisch mehrerer Phosphite der Formel eingesetzt werden kann.

Das Übergangsmetall kann vor der Umsetzung aus geeigneten chemischen Verbindungen, beispielsweise durch Reduktion mit unedlen Metallen wie Zink, aus Salzen, wie Chloriden, erhalten werden.

Setzt man zur Herstellung der Übergangsmetallkomplexe eine ein Übergangsmetall enthaltende Verbindung ein, so kommen hierzu vorteilhaft Salze, wie Chloride, Bromide, Acetylacetonate, Sulfate, Nitrate, beispielsweise Nickel(2)-Chlorid, in Betracht

Nach der Umsetzung der ein Übergangsmetall enthaltenden Verbindung oder des Übergangsmetalls mit einem Phosphit der Formel I kann die Wertigkeit des Übergangsmetalls in dem Komplex mit geeigneten Oxidations- oder Reduktionsmitteln, beispielsweise unedlen Metallen, wie Zink, oder Wasserstoff in chemisch gebundener Form, wie Natriumborhydrid, oder in molekularer Form, oder elektrochemisch verändert werden.

In den Übergangsmetallkomplexen kann das molare Verhältnis von Übergangsmetall zu Phospit der Formel I im Bereich zwischen 1 und 6, vorzugsweise 2 bis 5, insbesondere 2, 3 oder 4 betragen.

Die Übergangsmetallkomplexe können frei von anderen Liganden als den Phosphiten der Formel 1 sein.

Die Übergangsmetallkomplexe können neben den Phosphiten der Formel I weitere Liganden enthalten, beispielsweise Nitrile, wie Acetonitril, Adipodinitril, 3-Peniennitril, 4-Pentennitril, 2-Methyl-3-butennitril, Olefine, wie Butadien.

Die Herstellung solcher Übergangsmetallkomplexe kann grundsätzlich in einer solchen Weise erfolgen, wie sie in der Literatur, beispielsweise in DE-OS-2 237 703, US-A-3.850,973, US-A-3,766,237 oder US-A-3,903,120, zur Herstellung von Übergangsmetallkomplexen, die Tri-o-tolyl-phosphit, Tri-m-tolyl-phosphit oder Tri-p-tolyl-phosphit enthalten, beschrieben ist, indem man diese Phosphite durch die erfindungsgemäßen Phosphite der Formel I teilweise oder vollständig ersetzt.

Die erfindungsgemäßen Übergangsmetallkomplexe können als Katalysator, insbesondere als Homogenkatalysator eingesetzt werden.

Als besonders vorteilhaft hat sich die Verwendung der erfindungsgemäßen Übergangsmetallkomplexe als Katalysator bei der Addition von Blausäure an olefinische Doppelbindungen, insbesondere solche, die in Konjugation zu einer weiteren olefinischen Doppelbindung stehen, beispielsweise von Butadien unter Erhalt einer Mischung enthaltend 2-Methyl-3-butennitril und 3-Pentennitril, erwiesen. Gleichermaßen vorteilhaft ist auch die Verwendung als Katalysator bei der Addition von Blausäure an olefinische Doppelbindungen, die nicht in Verbindung mit einer weiteren olefinischen Doppelbindung stehen, beispielsweise von 3-Pentennitril oder 4-Pentennitril oder deren Gemische, vorzugsweise 3-Pentennitril, unter Erhalt von Adipodinitril, oder von 3-Pentensäureester oder 4-Pentensäureester oder deren Gemische, vorzugsweise 3-Pentensäureester, unter Erhalt von 5-Cyanovaleriansäureester.

Ebenso als besonders vorteilhaft hat sich die Verwendung der erfindungsgemäßen Übergangsmetallkomplexe als Katalysator bei der Isomerisierung organischer Nitrite, insbesondere solcher, bei denen die Nitrilgruppe nicht in Konjugation zu einer olefinischen Doppelbindung steht, beispielsweise von 2-Methyl-3-butennitril unter Erhalt von 3-Pentennitril, erwiesen. Gleichermaßen vorteilhaft ist auch die Verwendung als Katalysator bei der Isomerisierung organischer Nitrile, bei denen die Nitrilgruppe in Konjugation zu einer olefinischen Doppelbindung steht.

Verfahren zur Addition von Blausäure an eine olefinische Doppelbindung oder zur Isomerisierung von organischen Nitrilen kann grundsätzlich in einer solchen Weise erfolgen, wie sie in der Literatur unter Verwendung von Übergangsmetallkomplexen, die Tri-o-toiyl-phosphit, Tri-m-tolyl-phosphit oder Tri-p-tolyl-phosphit enthalten, beschrieben ist, indem man diese Phosphite durch die erfindungsgemäßen Phosphite der Formel teilweise oder vollständig ersetzt.

In diesen Verfahren weisen die erfindungsgemäßen Übergangsmetallkomplexe gegenüber solchen, die Tri-o-tolyl-phosphit als Ligand enthalten, eine erhöhte Stabilität und gegenüber solchen, die Tri-m/p-tolyl-phosphit als Ligand enthalten, eine erhöhte Reaktivität auf.

### Beispiele

### Beispiel 1 bis 4

Eine Lösung aus CIP(O-m-tol)₂ (298 g, 1.06 mol) in n-Hexan (2.5 I) wurde auf 0°C gekühlt (Eisbad). Aus zwei Tropftrichtern wurden Triethylamin (118 g, 1.17 mol) und 2-Isopropylphenol (1.06 mol) parallel innerhalb von 2 h bei 0 - 5°C zugegeben. Die Reaktionsmischung wurde bei Raumtemperatur 12 h gerührt Das ausgefallene NEt₃·HCl wurde mit einer Druckrlutsche abfiltriert und mit n-Hexan (250 ml) gewaschen. Dieser Ansatz wurde in ähnlicher Größe wiederholt (1.015 mol). Beide Rohlösungen wurden vereinigt und zur Entfernung von Restmengen an Chlor über eine Säule, gefüllt mit Al₂O₃, filtriert. Nach Einengen am Rotationsverdampfer (16 mbar, 50°C) wurde P(O-m-tol)₂(O-o-iPr-C₆H₄) (L1) erhalten; Ausbeute 94 %), ³¹P NMR (CDCl₃)δ130.1.

Die Liganden L2 - L4 wurden analog L1 hergestellt. Alle Ergebnisse sind in Tabelle 1 zusammengefasst.

**Tabelle 1: Beispiele 1 - 4**

| Beispiel | Edukte | Ligand | Ausbeute (%) | ³¹P NMRö (CDCl₃) |
|---|---|---|---|---|
| 1 | CIP(O-m-tol)₂, 2-Isopropylphenol | P(O-m-tol)₂(O-o-iPr-C₆H₄)L3 | 94 | 130.1 |
| 2 | CIP(P-m-tol)₂, 2-Secbutylphenol | P(O-m-tol)₂(O-o-secBu-C₆H₄)L4 | 94 | 129.8 |
| 3 | CIP(O-m-tol)₂, 2-Tertbutylphenol | P(O-m-tol)₂(O-o-tBu-C₆H₄)L5 | 96 | 129.6 |
| 4 | CIP(O-m-tol)₂, 2-Phenylphenol | P(O-m-tol)₂(O-o-Ph-C₆H₄)L6 | 80 | 129.5 |

### Beispiele 5 - 7

Eine Mischung aus m-Kresol, p-Kresol und o-Isopropylphenol entsprechend der molaren Zusammensetzung in Tabelle 2 (insgesamt 18 mol) wurde in einem 41-Vierhalskolben vorgelegt. Die Mischung wurde unter Inertgas auf 70°C erwärmt, Innerhalb von 5 h wurde PCI₃ (824 g, 6 mol) zugetropft. Das entstehende HCI wurde über einen Waschturm entsorgt. Die Temperatur wurde auf 110°C erhöht Ein leichter Stickstoffstrom wurde unter fortgesetztem Rühren durch die Reaktionsmischung geleitet, bis die HCI-Entwicklung beendet war.

**Tabelle 2: Beispiele 5 - 7**

| Beispiel | Produkt | Edukte (mol%) | | |
|---|---|---|---|---|
| | | o-Isopropylphenol | m-Kresol | p-Kresol |
| 5 | L5 | 20 | 53 | 27 |
| 6 | L6 | 33 | 45 | 22 |
| 7 | L7 | 40 | 40 | 20 |

### Beispiel 8

Ligand L1 aus Beispiel 1 (358.4 g), 3-Pentennitril (94.4 g), Nickelpulver (28.2 g) und CIP(O-m-tol)₂ (1.43 g) wurden unter Inertgas 25 h bei 95°C gerührt. Nach Abkühlen auf Raumtemperatur wurde überschüssiges Nickelpulver abfiltriert. Das Filtrat enthielt 1.28 Gew.-% Ni(O). Das Filtrat wurde mit 3-Pentennitril und zusätzlichem Ligand auf ein Ligand:Nickel(O) Verhältnis von 18:1 und eine Nickel(O)-Konzentration von 0.7 Gew.-% Ni(O) verdünnt. Diese Lösung wurde als Katalysatorlösung (K1) in Beispiel 21 eingesetzt.

### Beispiel 9

Ligand L5 aus Beispiel 5 (780 g), 3-Pentennitril (206 g), Nickelpulver (60 g), CIP(O-m-tol)₂ (3.3 g) und PCI₃ (35 Tropfen) wurden unter Inertgas 21 h bei 95°C gerührt Nach Abkühlen auf Raumtemperatur wurde überschüssiges Nickelpulver abfiltriert. Das Filtrat enthielt 1.5 Gew.-% Ni(O). Das Filtrat wurde mit 3-Pentennitril und zusätzlichem Ligand auf ein Ligand: Nickel(O) Verhältnis von 18:1 und eine Nickel(O)-Konzen-tration von 0.7 Gew.-% (Ni(O) verdünnt. Diese Lösung wurde als Katalysatorlösung (K2) in Beispiel 22 eingesetzt.

### Beispiel 10

Ligand L6 aus Beispiel 6 (780 g), 3-Pentennitril (206 g), Nickelpulver (60 g), CIP(O-m-tol)₂ (3,3 g) und PCI₃ (35 Tropfen) wurden unter Inertgas 29 h bei 95°C gerührt. Nach Abkühlen auf Raumtemperatur wurde überschüssiges Nickelpulver abfiltriert. Das Filtrat enthielt 1.1 Gew.-% Ni(O). Das Filtrat wurde mit 3-Pentennitril und zusätzlichem Ligand auf ein Ligand:Nicket(O) Verhältnis von 18:1 und eine Nickel(O)-Konzentration von 0.7 Gew.-% (Ni(O) verdünnt. Diese Lösung wurde als Katalysatorlösung (K3) in Beispiel 23 eingesetzt.

### Beispiel 11

Ligand L7 aus Beispiel 7 (500 g), 3-Pentennitril (132 g), Nickelpulver (40 g), CIP(O-m-tol)₂ (2.1 g) und PCI₃ (23 Tropfen) wurden unter Inertgas 23 h bei 95°C gerührt. Eine Probe enthielt 0.72 Gew.-% Ni(O). Weitere 20 g Nickelpulver sowie 1 g CIP(O-m-tol)₂ wurden zugegeben, und es wurde nochmals 18 h bei 95°C gerührt. Nach Abkühlen auf Raumtemperatur wurde überschüssiges Nickelpulver abfiltriert. Das Filtrat enthielt 0.77 Gew.-% Ni(O). Das Filtrat wurde mit 3-Pentennitril und zusätzlichem Ligand auf ein Ligand:Nickel(O) Verhältnis von 18:1 und eine Nickel(O)-Konzentration von 0,6 Gew.-% Ni(O) verdünnt. Diese Lösung wurde als Katalysatorlösung (K4) in Beispiel 24 eingesetzt.

### Beispiele 12 -15

Jeweils 50 mmol 2-Methyl-3-butennitril (2M3BN) wurden mit Katalysatorlösung K1-4 aus den Beispielen 8-11 (0.2 mmol Ni) 2 h bei 130°C umgesetzt. Um Zerfallsprozesse durch Luft und Feuchtigkeit auszuschließen, wurden die Reaktionen in einem geschlossenen System durchgeführt. Umsatz und Selektivität wurden nach Ende der Reaktion per GC bestimmt. Zum Vergleich wurde unter identischen Bedingungen (50 mmcl 2M3BN, 0.2 mmol Ni, 130°C, 2 h) eine Ni(m-/p-Tolylphosphit-Komplexlösung (m/p-Tolylphosphit:Ni = 18:1, 0.7 Gew.-% Ni(O), Herstellung der Komplexlösung analog zu K1-6 aus m/p-Tolylphosphit und Nickelpulver in Gegenwart von 3-Pentennitril (Vergleichsbeispiel A) eingesetzt.

**Tabelle 3: Beispiele 12-15**

| Beispiel | Katalysatorlösung | Umsatz (%) | Selektivität (c/t-3PN in %) |
|---|---|---|---|
| 12 | K1 | 81.7 | 92.4 |
| 13 | K2 | 29.0 | 95.7 |
| 14 | K3 | 56.0 | 97.7 |
| 215 | K4 | 78.3 | 97.8 |
| Vgl. A | Ni(m/p-Tolylphosphit) ohne ZnCl₂ | 7.9 | 81.7 |

## Patentansprüche

1. Phosphite der Formell
P (O-R¹)ₓ (O-R²)_{y} (O-R³)_{z} (O-R⁴)ₚ I
mit
R¹: aromatischer Rest mit einem Alkylsubstituenten aus der Gruppe n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können, in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet,
R²: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anelüerten aromatischen System, wobei der aromatische Rest in o-Stellung zu dem Sauerstoffatom, das das Phosphoratom mit dem aromatischen System verbindet, ein Wasserstoffatom trägt,
R³: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,
R⁴: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
x : 1 oder 2,
y, z, p: unabhängig voneinander 0, 1 oder 2 mit der Maßgabe, dass x+y+z+p = 3.

2. Phosphit der Formel I nach Anspruch 1 mit p = 0.

3. Phosphit der Formel I nach den Ansprüchen 1 bis 2, wobei die Reste R¹, R², R³ und R⁴ substituierte oder unsubstituierte Phenylreste sind.

4. Verfahren zur Herstellung eines Phosphits der Formel I gemäß den Ansprüchen 1 bis3, **dadurch gekennzeichnet, daß** man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I.

5. Verwendung eines Phosphits I gemäß den Ansprüchen 1 bis 3 als Ligand in Übergangsmetallkomplexen.

6. Übergangsmetallkomplexe enthaltend als Ligand ein Phosphit I gemäß den Ansprüchen 1 bis 3.

7. Übergangsmetallkomplexe nach Anspruch 6, wobei man als Übergangsmetall Nickel einsetzt.

8. Verfahren zur Herstellung von Übergangsmetallkomplexen gemäß Anspruch 6 oder 7, **dadurch gekennzeichnet, dass** man ein elementares Übergangsmetall oder eine ein Übergangsmetall enthaltende chemische Verbindung mit einem Phosphit der Formel I umsetzt.

9. Verwendung von Übergangsmetallkomplexen gemäß Anspruch 6 oder 7 als Katalysator.

10. Verwendung nach Anspruch 9 als Katalysator für die Addition von Blausäure an eine olefinische wppelbindung.

11. Verwendung nach Anspruch 9 als Katatysatorfür die Isomerisierung organischer Nitrile.

12. Verfahren zur Addition von Blausäure an eine olefinische Doppelbindung in Gegenwart eines Katalysators, wobei man als Katalysator einen Übergangsmetallkomplex gemäß Anspruch 6 oder 7 einsetzt.

13. Verfahren nach Anspruch 12, wobei man Blausäure an Butadien addiert unter Erhalt einer Verbindung ausgewählt aus der Gruppe bestehend aus 2-Methyl-3-butennitril und 3-Pentennitril.

14. Verfahren zur Isomerisierung organischer Nitrile in Gegenwart eines Katalysators, wobei man als Katalysator einen Übergangsmetallkomplex gemäß Anspruch 6 oder 7 einsetzt.

15. Verfahren nach Anspruch 14, wobei man 2-Methyl-3-butennitril zu 3-Pentennitril isomerisiert.

## Claims

1. A phosphite of the formula I
P (O-R¹)ₓ (O-R²)_{y} (O-R³)_{z} (O-R⁴)ₚ I
where
R¹: aromatic radical having an alkyl substituent from the group consisting of n-propyl, i-propyl, n-butyl, i-butyl, s-butyl, n-pentyl and its isomers, n-hexyl and its isomers, cyclopentyl and cyclohexyl radicals, where the cyclic alkyl radicals may bear linear or further cyclic alkyl radicals or aromatic radicals as substituents and the alkyl radicals may bear cyclic alkyl radicals or aromatic radicals as substituents, in the o position relative to the oxygen atom which connects the phosphorus atom to the aromatic system, or having an aromatic substituent in the o position relative to the oxygen atom which connects the phosphorus atom to the aromatic system, or having an aromatic system fused on in the o position relative to the oxygen atom which connects the phosphorus atom to the aromatic system,
R²: aromatic radical having a C₁-C₁₈-alkyl substituent in the m position relative to the oxygen atom which connects the phosphorus atom to the aromatic system, or having an aromatic substituent in the m position relative to the oxygen atom which connects the phosphorus atom to the aromatic system, or having an aromatic system fused on in the m position relative to the oxygen atom which connects the phosphorus atom to the aromatic system, where the aromatic radical bears a hydrogen atom in the o position relative to the oxygen atom which connects the phosphorus atom to the aromatic system,
R³: aromatic radical having a C₁-C₁₈-alkyl substituent in the p position relative to the oxygen atom which connects the phosphorus atom to the aromatic system, or having an aromatic substituent in the p position relative to the oxygen atom which connects the phosphorus atom to the aromatic system, where the aromatic radical bears a hydrogen atom in the o position relative to the oxygen atom which connects the phosphorus atom to the aromatic system,
R⁴: aromatic radical which bears substituents other than those defined for R¹, R² and R³ in the o, m and p positions relative to the oxygen atom which connects the phosphorus atom to the aromatic system, where the aromatic radical bears a hydrogen atom in the o position relative to the oxygen atom which connects the phosphorus atom to the aromatic system,
x : 1 or 2,
y, z, p: independently of one another, 0, 1 or 2, with the proviso that x+y+z+p = 3.

2. The phosphite of the formula I according to claim 1 in which p = 0.

3. The phosphite of the formula I according to claim 1 or 2, wherein the radicals R¹, R², R³ and R⁴ are substituted or unsubstituted phenyl radicals.

4. A process for preparing a phosphite of the formula I according to any of claims 1 to 3, which comprises
a) reacting a phosphorus trihalide with an alcohol selected from the group consisting of R¹OH, R²OH, R³OH and R⁴OH and mixtures thereof to give a dihalophosphorous monoester,
b) reacting this dihalophosphorous monoester with an alcohol selected from the group consisting of R¹OH, R²OH, R³OH and R⁴OH and mixtures thereof to give a monohalophosphorous diester and
c) reacting this monohalophosphorous diester with an alcohol selected from the group consisting of R¹OH, R²OH, R³OH and R⁴OH and mixtures thereof to give a phosphite of the formula I.

5. The use of a phosphite I according to any of claims 1 to 3 as ligand in transition metal complexes.

6. A transition metal complex comprising a phosphite I according to any of claims 1 to 3 as ligand.

7. The transition metal complex according to claim 6, wherein nickel is used as transition metal.

8. A process for preparing transition metal complexes according to claim 6 or 7, which comprises reacting an elemental transition metal or a chemical compound comprising a transition metal with a phosphite of the formula I.

9. The use of transition metal complexes according to claim 6 or 7 as catalyst.

10. The use according to claim 9 as catalyst for the addition of hydrocyanic acid onto an olefinic double bond.

11. The use according to claim 9 as catalyst for the isomerization of organic nitriles.

12. A process for the addition of hydrocyanic acid onto an olefinic double bond in the presence of a transition metal complex according to claim 6 or 7 as catalyst.

13. The process according to claim 12, wherein hydrocyanic acid is added onto butadiene to give a compound selected from the group consisting of 2-methyl-3-butenenitrile and 3-pentenenitrile.

14. A process for the isomerization of organic nitriles in the presence of a transition metal complex according to claim 6 or 7 as catalyst.

15. The process according to claim 14, wherein 2-methyl-3-butenenitrile is isomerized to 3-pentenenitrile.

## Revendications

1. Phosphite de formule
P(O-R¹)ₓ(O-R²)_{y}(O-R³)_{z}(O-R⁴)ₚ I
avec :
R¹ : reste aromatique comportant un substituant alkyle du groupe formé par des radicaux n-propyle, i-propyle, n-butyle, i-butyle, s-butyle, n-pentyle ainsi que leurs isomères, des radicaux n-hexyle ainsi que leurs isomères, des radicaux cyclopentyle ou cyclohexyle, les radicaux alkyle cycliques pouvant porter en tant que substituants des radicaux alkyle linéaires ou d'autres radicaux alkyle cycliques ou des radicaux aromatiques, et les radicaux alkyle des radicaux alkyle cycliques ou des radicaux aromatiques, en position ortho par rapport à l'atome d'oxygène qui relie l'atome de phosphore au système aromatique, ou un substituant aromatique en position ortho par rapport à l'atome d'oxygène qui relie l'atome de phosphore au système aromatique;
R² : reste aromatique comportant un substituant alkyle en C₁ à C₁₈ en position méta par rapport à l'atome d'oxygène qui relie l'atome de phosphore au système aromatique, ou un substituant aromatique en position méta par rapport à l'atome d'oxygène qui relie l'atome de phosphore au système aromatique, ou un système aromatique annélé, en position méta par rapport à l'atome d'oxygène qui relie l'atome de phosphore au système aromatique, le reste aromatique portant un atome d'hydrogène en position ortho par rapport à l'atome d'oxygène qui relie l'atome de phosphore au système aromatique;
R³ : reste aromatique comportant un substituant alkyle en C₁ à C₁₈ en position para par rapport à l'atome d'oxygène qui relie l'atome de phosphore au système aromatique, ou un substituant aromatique en position para par rapport à l'atome d'oxygène qui relie l'atome de phosphore au système aromatique, le reste aromatique portant un atome d'hydrogène en position ortho par rapport à l'atome d'oxygène qui relie l'atome de phosphore au système aromatique;
R⁴ : reste aromatique portant, en position ortho, méta et para par rapport à l'atome d'oxygène qui relie l'atome de phosphore au système aromatique, d'autres substituants que ceux définis pour R¹, R² et R³, le reste aromatique portant un atome d'hydrogène en position ortho par rapport à l'atome d'oxygène qui relie l'atome de phosphore au système aromatique;
x : 1 ou 2;
y, z, p : indépendamment l'un de l' autre, 0, 1 ou 2, à la condition que x + y + z + p = 3.

2. Phosphite de la formule I selon la revendication 1, dans lequel p = 0.

3. Phosphite de la formule I selon les revendications 1 à 2, dans lequel les restes R¹, R², R³ et R⁴ sont des radicaux phényle substitués ou non substitués.

4. Procédé de préparation d'un phosphite de la formule I selon les revendications 1 à 3, **caractérisé en ce que** :
a) on fait réagir un trihalogénure de phosphore avec un alcool choisi dans le groupe formé par R¹OH, R²OH, R³OH et R⁴OH ou leurs mélanges, avec obtention d'un monoester d'acide dihalogénophosphoreux,
b) on fait réagir ledit monoester d'acide dihalogénophosphoreux avec un alcool choisi dans le groupe formé par R¹OH, R²OH, R³OH et R⁴OH ou leurs mélanges, avec obtention d'un diester d'acide monohalogénophosphoreux,
c) on fait réagir ledit diester d'acide monohalogénophosphoreux avec un alcool choisi dans le groupe formé par R¹OH, R²OH, R³OH et R⁴OH ou leurs mélanges, avec obtention d'un phosphite de la formule I.

5. Utilisation d'un phosphite I selon les revendications 1 à 3 en tant que ligand dans des complexes de métaux de transition.

6. Complexes de métaux de transition contenant en tant que ligand un phosphite I selon les revendications 1 à 3.

7. Complexes de métaux de transition selon la revendication 6, dans lequel on met en oeuvre du nickel en tant que métal de transition.

8. Procédé de préparation de complexes de métaux de transition selon la revendication 6 ou 7, **caractérisé en ce que** l'on fait réagir un métal de transition élémentaire ou un composé chimique contenant un métal de transition, avec un phosphite de la formule I.

9. Utilisation de complexes de métaux de transition selon la revendication 6 ou 7 en tant que catalyseurs.

10. Utilisation selon la revendication 9 en tant que catalyseurs pour l'addition d'acide cyanhydrique sur une double liaison oléfinique.

11. Utilisation selon la revendication 9 en tant que catalyseurs pour l'isomérisation de nitriles organiques.

12. Procédé d'addition d'acide cyanhydrique sur une double liaison oléfinique en présence d'un catalyseur, où l'on met en oeuvre en tant que catalyseur un complexe de métal de transition selon la revendication 6 ou 7.

13. Procédé selon la revendication 12, dans lequel on ajoute de l'acide cyanhydrique sur du butadiène, avec obtention d'un composé choisi dans le groupe formé par le 2-méthyl-3-butènenitrile et le 3-pentènenitrile.

14. Procédé d'isomérisation de nitriles organiques en présence d'un catalyseur, dans lequel on met en oeuvre en tant que catalyseur un complexe de métal de transition selon la revendication 6 ou 7.

15. Procédé selon la revendication 14, dans lequel on isomérise du 2-méthyl-3-butènenitrile en 3-pentènenitrile.
